# EUROPEAN PATENT APPLICATION

(11) **EP 3 643 332 A1**
(43) Date of publication of application: **29.04.2020**
(21) Application number: 17913329.3
(22) Date of filing: 06.07.2017
(51) Int. Cl.: A61L 27/36, A61L 27/24

(54) **PREPARATION METHOD AND USAGE METHOD FOR CARTILAGE TISSUE RECOVERY COLLAGEN**

(30) Priority: 15.06.2017 KR 20170076098
(71) Applicant: Sewoncellontech Co., Ltd., Seoul 07325 (KR)
(72) Inventor: LEE, Joon Ho, Seoul 04781 (KR); YOO, Ji Chul, Namyangju-si Gyeonggi-do 12095 (KR); SUH, Dong Sam, Seoul 05837 (KR); CHANG, Cheong Ho, Seoul 06195 (KR)
(74) Representative: dompatent von Kreisler Selting Werner - Partnerschaft von Patent- und Rechtsanwälten mbB
(86) International application number: PCT/KR2017/007220
(87) International publication number: WO 2018/230765

(57) **Abstract**

The present invention relates to: a method for preparing a cartilage tissue recovery collagen usable for a joint in an injection manner; and a usage method therefor. To this end, the present invention provides: a method for obtaining a high-concentration collagen solution injection by aseptically filling an injectable container with collagen isolated from pig skin tissue; and a method for using a high-viscosity collagen, which is filled in the injectable container, for the purpose of cartilage recovery. It was ascertained in the present invention that cartilage tissue regeneration is effectively induced when tissue recovery is implemented with respect to a cartilage defect site by using collagen, which is a biocompatible material, in a form injectable into an application site with a needle without a surgical incision. Resultantly, the present invention allows cartilage recovery and regeneration to be easily and quickly induced in an animal, excluding human, while relieving a surgery-related burden, and thus the quality and the reliability of a product are greatly improved such that a good image can be provided by satisfying the various needs of consumers which are users.

## Description

### Technical Field

The present invention relates to a method of manufacturing collagen for restoring cartilage tissue and a usage method thereof. More particularly, in the present invention, collagen, which is a biocompatible material, is manufactured in an injectable form that is capable of being grafted onto a cartilage-deficient portion. The collagen is directly injected to an application site using an injection needle without a surgical incision to thus restore tissue, so that the regeneration of cartilage tissue is effectively induced. Accordingly, the restoration and regeneration of cartilage are easily and quickly induced in an animal, excluding a human, while relieving the burden related to surgery. Therefore, the quality and the reliability of a product are greatly improved, thus satisfying the various needs of consumers, that is, users, thereby providing a good image.

### Background Art

Cartilage is a tissue that is not regenerated *in vivo* once damaged. Cartilage damage is accompanied by pain and limited range of motion, and eventually proceeds to degenerative arthritis, which is a disease in which cartilage is worn out and local degenerative changes appear. Degenerative arthritis afflicted as many as 60 million patients worldwide in 2010, and the arthritis-related market is increasing at an average rate of 14% per year. About 80% of people over 55 suffer from degenerative arthritis. Moreover, young people are also at risk of injury due to increased interest and participation in cultural activities such as leisure sports and extreme sports. According to the Statistics Korea, in Korea, the proportion of the population age 65 or older is expected to reach 10.7% in 2009, 14.3% in 2018, and 20.8% in 2026, and Korea is thus becoming a 'super-aged society'. In countries with a large proportion of elderly persons, the proportion of patients with arthritis will grow even higher, and arthritis is considered a disease that can affect anyone. Accordingly, arthritis is a disease of great interest in health and medical policy. In addition, elderly patients suffering from arthritis urgently require a surgical treatment using a minimally invasive method (a method for minimizing the scale of an operation) that incurs less physical and economic burden.

Examples of a conventional method for treating damaged cartilage include cartilage debridement, guided bone marrow regeneration (bone marrow stimulating technique), autochondral grafting surgery (osteochondral autograft), and autochondral cell grafting surgery depending on the damage to the cartilage. However, these methods mostly require an invasive operation to repair very damaged cartilage. Recently, as a treatment method applicable to early stages of cartilage damage, a method of injecting a hyaluronic acid product into a glenoid cavity has been extensively used. However, the hyaluronic acid product injected into the joint serves as a component similar to a synovial component to thus replace only the synovial component with a curing agent (medicine), thereby simply alleviating pain through a lubrication action. On the contrary, injecting purified collagen having ideal viscosity for flexible movement of joints may replace the synovial component to thus relieve pain through a lubrication action and may also form a flexible network with surrounding tissue, thereby coating sites of damaged biological tissue (cartilage) therewith. Further, injection of the purified collagen may promote cell infiltration and angiogenesis, thus helping regeneration of the lamina splendens and tissue membranes and strengthening intra-articular tissue.

Collagen is a major protein constituting cartilage, ligaments, tendons, and bones, which are human tissues and organs, and is a structural protein that accounts for 25 to 30% of the protein constituting the human body. In order to regenerate tissue, collagen acts as a basic material constituting the tissue, provides space for cell proliferation to thus activate cell growth and cytodifferentiation, and stimulates the platelets in the blood to thus secrete growth factors. Collagen is therefore an essential protein that is essential *in vivo* for the regeneration of tissues through interactions with cellular and blood components.

### Documents of Related Art

(Patent Document 1) Korean Patent No. 10-1279812 (2013. 6. 28)
(Patent Document 2) Korean Patent No. 10-1114773 (2012. 3. 5)
(Patent Document 3) Korean Patent No. 10-0684932 (2007. 2. 20)
(Patent Document 4) Korean Laid-Open Patent Application No. 2004-0008125 (2004. 1. 28)

### Disclosure

### Technical Problem

Examples of a conventional method for treating damaged cartilage include cartilage debridement, guided bone marrow regeneration (bone marrow stimulating technique), autochondral grafting surgery (osteochondral autograft), and autochondral cell grafting surgery, depending on the damage to the cartilage. These treatment methods have problems such as requiring resection for operation, extraction of the periosteum, complexity of use, expensive treatment costs, leakage of adult stem cells induced in the process of stimulating bone marrow, and generation of abnormal fibrocartilage due to hemostasis problems. Therefore, in order to solve the above-described problems, there is urgent need for an operation using a minimally invasive method or a medical treatment to replace an operation using an invasive method.

The present invention provides a method of manufacturing collagen for restoring cartilage tissue and a usage method thereof. The collagen is injected to protect and reinforce articular cartilage, so that a membrane covering the tissue of the articular cartilage is coated with the collagen to thus protect the tissue, thereby curing osteoarthritis and preventing cartilage damage.

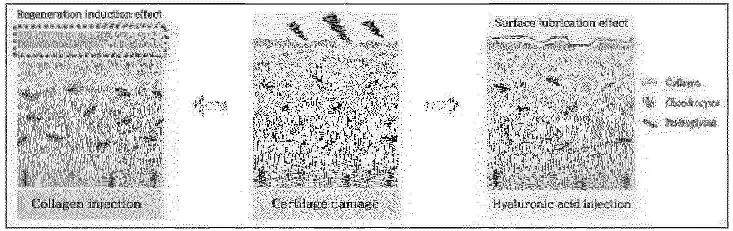

### <Comparison of concepts of collagen and hyaluronic acid injection treatment>

### Technical Solution

The present invention provides a method of manufacturing collagen for restoring connective tissue, in which a pig skin tissue is fragmentized, washed, and subjected to enzyme treatment, a collagen is separated therefrom, and high-concentration collagen aseptically loaded into an injection container is obtained through salt treatment, sterilization by filtration, concentration, and loading processes. The method includes a process of separating the collagen from the skin tissue and loading the collagen into the injection container. The process includes washing the skin tissue separated from a pig using ethanol (at least 70%, 20 to 24 hrs) and sodium hydroxide (pH of 11 or more, 2 hrs), followed by fragmentizing; mixing the fragmentized tissue with an acidic solution (pH of 3 or less) containing an enzyme, followed by agitation at 30°C or less for 3 to 5 days to perform a reaction; adding NaCl in order to obtain the collagen separated from the tissue so that a final concentration is 0.7 to 0.9M; performing passing through a membrane filter to separate materials (non-collagen protein, salt, pepsin, etc.) having a molecular weight that is smaller than a collagen molecular weight (300 kDa), followed by passing through a filter having pores of 0.22 µm to obtain the sterile collagen; performing agglomeration of the collagen using a pH and a temperature, followed by concentration via centrifugation; and filling the injection container with the concentrated collagen using sterile filling tools.

### Advantageous Effects

As described in detail above, the present invention provides a composition for restoring cartilage tissue obtained by aseptically filling a syringe with collagen at a diluted concentration of 5 to 60 mg/mL except water or a physiological phosphate buffer solution.

In the present invention, in order to overcome the drawback (simple lubrication action) of a conventional joint injection including a hyaluronic acid component, collagen, which is a biocompatible material, is used to restore damaged cartilage.

When the collagen is grafted onto a cartilage-deficient portion, collagen fibers form a flexible network with surrounding tissues so that wounds of damaged biological tissues are coated therewith, thus promoting cell infiltration and angiogenesis, which helps the natural healing process. Further, the intra-articular tissue membrane is strengthened to protect the joint tissue and increase the elasticity of the joint tissue, which helps the action of lubricating the joint tissue, thereby strengthening the function of the joint tissue.

Further, in the present invention, the collagen is manufactured so as to be applied in an injection manner using an aseptic operation method, which enables an operator to perform medical treatment using a non-invasive method. Accordingly, it is possible to simply and effectively stabilize a damaged cartilage tissue portion.

Moreover, it is possible to alleviate the pain caused by cartilage deficiencies and to promote the recovery of function of the joints and satisfaction with daily life.

### Description of Drawings

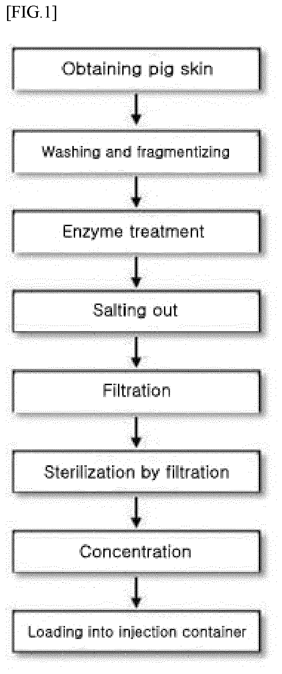

FIG. 1 shows a process of manufacturing a collagen solution aseptically loaded into an injection container;

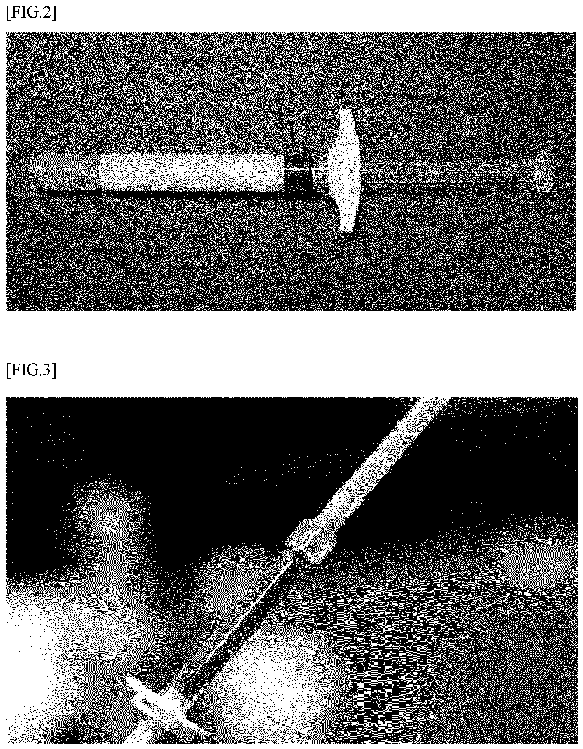

FIG. 2 shows the collagen solution aseptically loaded into the injection container;
FIG. 3 is a photograph showing the collagen solution that is stained blue in order to confirm the effect of injection into a cartilage portion;

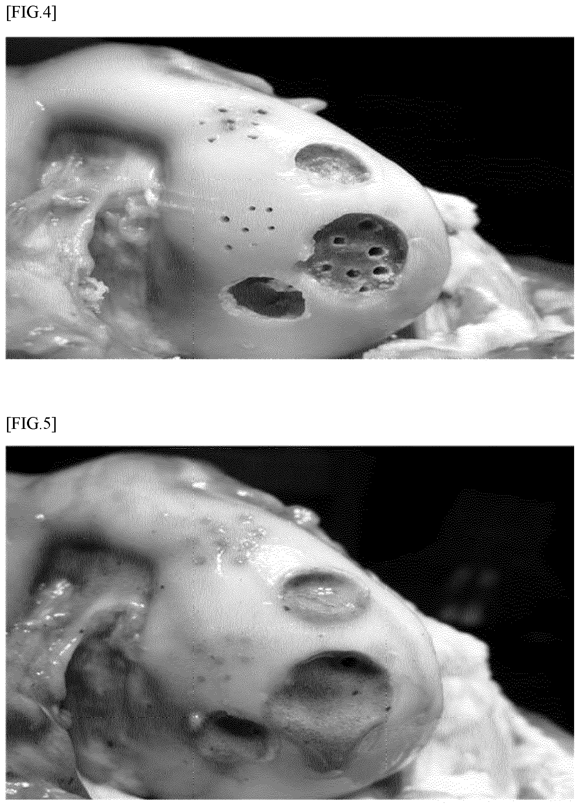

FIG. 4 is a photograph showing cartilage deficiency in a pig's knee induced in order to confirm the effect of the collagen for injection; and
FIG. 5 is a photograph showing the result of confirmation of the effect of injection of the collagen and the cartilage-deficient portion filled with the collagen.

### Mode for Invention

### (Example 1)

### Method of aseptically filling an injection container with collagen after manufacture of high-concentration collagen from which immune site is removed

A pig skin tissue is fragmentized, washed, and subjected to enzyme treatment, collagen is separated therefrom, and high-concentration collagen aseptically loaded into an injection container is obtained through salt treatment, sterilization by filtration, concentration, and filling processes.
1. The obtained pig skin tissue is fragmentized after washing with ethanol (at least 70%, 24 hr) and sodium hydroxide (pH of 11 or more, 2 hr)
2. The fragmentized tissue is mixed with an acidic solution containing an enzyme (pH of 3 or less), followed by agitation at 30°C or less for 3 to 5 days to perform a reaction.
3. NaCl is added thereto so that a final concentration is 0.7 to 0.9 M in order to obtain the collagen separated from the tissue.
4. Passing through a membrane filter is performed in order to separate materials (non-collagen protein, salt, pepsin, etc.) having a molecular weight that is smaller than a collagen molecular weight (300 kDa).
5. Passing through a filter having pores of 0.22 µm is performed in order to obtain sterile collagen.
6. Agglomeration of the collagen using a pH and a temperature is performed, followed by concentration via centrifugation.
7. The injection container is filled with the concentrated collagen solution using sterile filling tools.

### (Example 2)

### Method of applying the collagen loaded into an injection container to the cartilage-deficient portion of an animal

Purpose: To confirm the ability to apply collagen to the cartilage-deficient portion of a pig's knee in an injection manner

### <Method and Effect Confirmation>

1. A collagen solution (a concentration of 5 to 60 mg/mL) aseptically loaded into an injection container is prepared (collagen concentration). In order to realize cartilage restoration, which is the purpose of this product, it is required that the collagen concentration be 5 mg/mL or more so as to fill the deficient portion with the collagen and then maintain a predetermined form. Due to the characteristics (aseptic manufacture) of the process for manufacturing products for medical purposes (injection type), it difficult to manufacture, use in practice, and apply a product having a very high viscosity of 60 mg/mL or more. For this reason, the collagen concentration is set to the range of 5 mg/mL or more and 60 mg/mL or less.
2. The collagen solution is stained with a small amount of blue dye (Trypan blue, bonded to proteins) in order to confirm with the naked eye the effect after injection into the knee cartilage tissue of an animal (pig).
3. After a pig leg is fixed to a cradle to expose the cartilage of the knee joint using surgical devices, a drill is used to induce deficient portions having diameters of about 4 cm and depth of about 2 cm.
4. An injection needle is connected to the injection container filled with the collagen. An injection needle that is 38 mm or more long is used.
5. A cut portion is sutured using a suture thread, and the stained collagen contained in the injection container is directly injected into a glenoid cavity (space filled with synovia) of the pig's knee.
6. A knee joint motion (CPM: continuous passive motion) is performed to help the action of collagen naturally filling the deficient portion by injection. In addition, the sutured portion is cut to expose the cartilage portion into which the product is injected, and then is observed.

### <Result>

The collagen injected into the deficient portion of the cartilage naturally filled a deficiency-induced portion, and the effect desired by the operator was observed.

### (Example 3)

### Application method for treating the cartilage of animals using collagen loaded into injection container

Purpose: Test for confirming the cartilage curing effect of collagen loaded into an injection container using rabbits having deficient cartilage in joints thereof

### <Method and Effect Confirmation>

1. A preclinical experiment was performed using a rabbit (New Zealand white rabbit) in order to confirm the effect of curing the cartilage of the joint. The experimental rabbits were fasted for one day before the manufacture of a cartilage-deficient model and the surgical treatment for collagen injection.
2. The rabbits were anesthetized by injecting an anesthetic on the day of the surgical treatment, and the hair on a portion to be surgically treated was removed using a hair removal machine.
3. After the completion of hair removal, the portion to be surgically treated was cleaned with 70% ethanol and then disinfected with povidin.
4. After the knee joint skin of the rabbit was incised using a surgical operation device, the subcutaneous tissue was opened to expose the furrow portion of the patella of the articular cartilage.
5. A cartilage defect that was 2 mm in diameter and 2 mm in depth was induced using a surgical operation device.
6. The incised subcutaneous tissue and skin were sutured using a suture thread and then disinfected with povidin.
7. After an injection needle was placed in the injection container, collagen was injected into the cartilage-deficient portion.
8. After cartilage defects were induced in the legs of the experimental rabbits as a control group using the above-described method, physiological saline was injected thereto.
9. Antibiotics and analgesics were injected into the experimental rabbits, and the rabbits were placed in a rearing environment and checked the recovery from anesthesia.

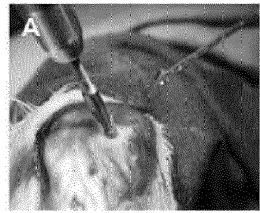

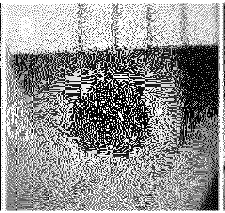

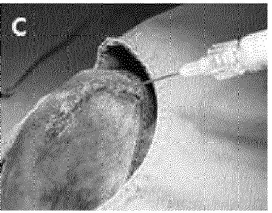

### <Photograph showing manufacture of cartilage-deficient model and surgical treatment of collagen injection>

A: Induction of cartilage defects, B: Size of cartilage defects (Ø 2 mm), C: Injection of collagen

10. The knee joint tissues of the experimental rabbits were collected at the expense of the experimental rabbits 3, 6, 9, and 12 weeks after the completion of the surgical treatment, and change patterns (change of cartilage-deficient portions, surface states, and continuity of the border between the tissues surrounding the defects and neoplastic tissues) were observed with the naked eye.
11. The tissues collected 3 and 12 weeks after the completion of the surgical treatment were fixed in 10% neutral formalin and were subjected to paraffin embedding to manufacture a 5 µm tissue specimen. Hematoxylin-Eosin, Safranin O, Toluidine blue, Type 1 collagen and Type 2 collagen staining methods were performed to observe the regeneration of cells and substrates.

### <Result>

The cartilage-deficient portion of the rabbit was observed with the naked eye 3, 6, 9, and 12 weeks after collagen and physiological saline were injected into the cartilage-deficient portion of the rabbit. As a result, it was confirmed that the deficient portion was restored after 3 weeks in the experimental group into which the collagen was injected. However, it was observed that the deficient portion was not properly restored even after 3 weeks in the control group. After that, it was confirmed that the cartilage surface was smooth because the deficient portion was restored in the experimental group into which the collagen was injected, but that the surface of the cartilage-deficient portion was not smooth in the control group into which the physiological saline was injected.

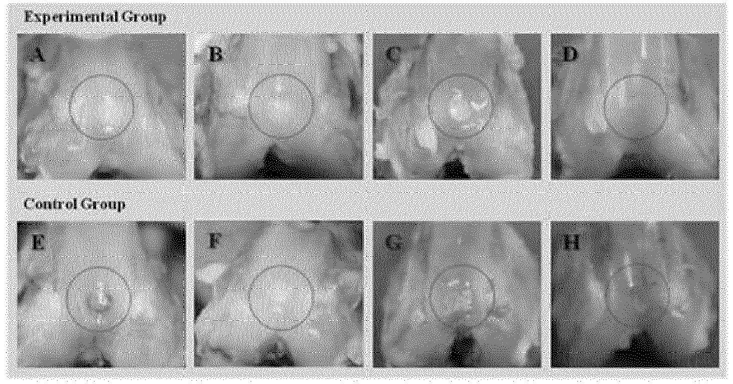

### <Observation with naked eye to confirm state of restoration of cartilage-deficient portion of rabbit>

Experimental group: A, B, C, and D, Control group: E, F, G, and H, Observation time point: 3 weeks (A, E), 6 weeks (B, F),
9 weeks (C, G), 12 weeks (D, H)

The cartilage of the rabbit, into which the collagen as the experimental group and the physiological saline as the control group were injected, was analyzed using tissue staining. As a result, it was confirmed that, in the case of the tissue into which the collagen was injected, a cartilage-deficient space was filled with the collagen after 3 weeks and the cartilage tissue was regenerated after 12 weeks. However, it was confirmed that the tissue into which the physiological saline was injected did not sufficiently fill the cartilage-deficient space after 3 weeks and the cartilage tissue was not sufficiently regenerated even after 12 weeks, and accordingly, the cartilage surface was not smooth.

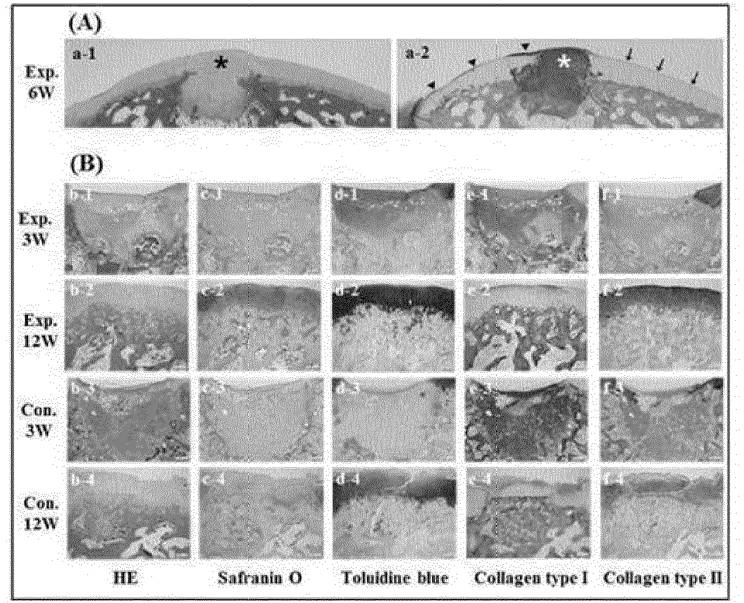

### <Histopathological analysis of rabbit cartilage tissue>

(A) Specimens of experimental groups at 6th week (a-1: H&E staining method, a-2: type 1 collagen staining method)
(B) Specimens of experimental and control groups at 3rd and 12th weeks (b: H&E staining method, c: Safranin-O staining method,
   d: Toluidine Blue staining method, e: type 1 collagen staining method, f: type 2 collagen staining method,
   1: Experimental group at 3rd week, 2: Experimental group at 12th week, 3: Control group at 3rd week, and 4: Control group at 12th week)

### (Example 4)

### Method using collagen loaded into injection container applied to treat patient suffering from knee arthritis

Purpose: Test to confirm whether pain is relieved or the quality of life is improved after injecting collagen loaded into an injection container into the knee of a patient suffering from chondromalacia or degenerative arthritis

### <Method and Effect Confirmation>

1. Two hundred patients suffering from chondromalacia or degenerative arthritis in the knees thereof were recruited.
2. The patients that were recruited were randomly divided into an experimental group (group in which collagen was injected into the knee joint, BioCollagen group) and a control group (group in which saline was injected into the knee joint, placebo group) to perform surgical treatment.
3. Patients were assessed for pain relief (VAS*), joint motion function improvement (WOMAC**), and daily life satisfaction (SF-36***) 4, 12 and 24 weeks after the completion of surgical treatment.
   * VAS (Visual Analogue Scale): A method of quantifying the pain level of a patient. The lower the numerical value, the less pain.
   ** WOMAC (Western Ontario and McMaster Universities Arthritis Index): A method of evaluating functional aspects of joint motion throughout a patient's daily life with respect to pain of the knee joint. As evaluation items, pain, stiffness, and the quality of life were measured and compared. The lower the numerical value, the better the joint's function.
   *** SF-36 (36-Item Short-form Health Survey): A method of measuring a patient's daily life satisfaction. The higher the numerical value, the higher the patient's satisfaction.

### <Result>

As a result of measurement and analysis using the VAS method, it was confirmed that, in the experimental group (group into which collagen was injected), the pain was relieved to 60.51 ± 13.82 mm before curing, but after 6 months, the pain was relieved to 28.88 ± 24.77 mm (A). Further, among the patients of the experimental group, 65 patients (73.86%) had a 20% improvement in VAS results and 59 patients (67.05%) had a 40% improvement. On the contrary, among the patients of the control group, 46 patients (54.12%) had a 20% improvement in VAS results and 38 patients (44.71%) had a 40% improvement (B).

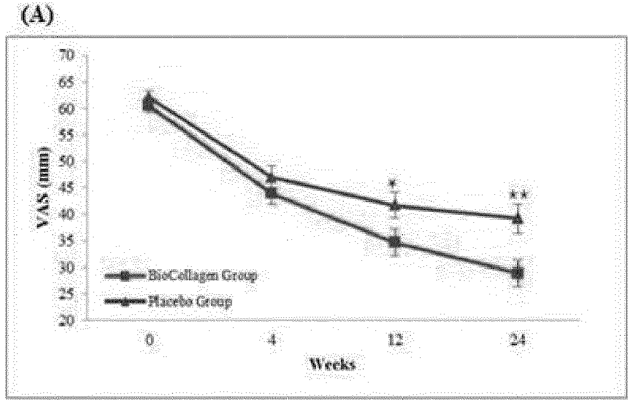

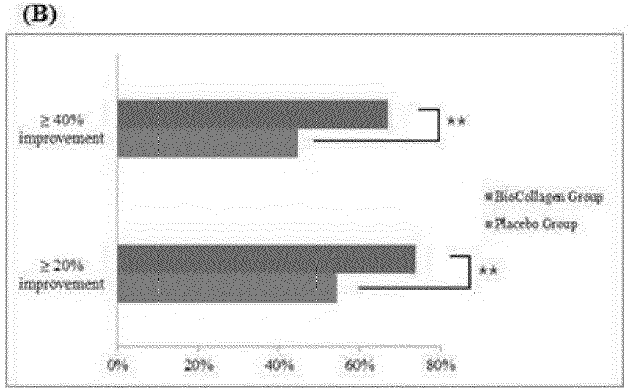

### <Results of analysis of treatment effect on experimental and control groups using VAS method>

(A) 100 mm VAS analysis result, (B) Pain relief rate of the patient
As a result of measurement and analysis using a VAS method, it was confirmed that, in the experimental group (group into which collagen was injected), the pain was relieved to 16.17 ± 18.18 after 4 weeks, 23.50 ± 24.88 after 12 weeks, and 28.40 ± 27.38 after 24 weeks in comparison with the case before curing, thereby verifying the pain relieving effect (A). As a result of measurement and analysis using a WOMAC method, it was confirmed that, in the experimental group (group into which collagen was injected), the pain was relieved to 9.90 ± 13.62 after 4 weeks, 13.20 ± 15.96 after 12 weeks, and 13.46 ± 17.45 after 24 weeks in comparison with the case before curing, thus relieving pain and improving stiffness and the quality of life (B). In addition, as a result of measurement and analysis using a SF-36 method, it was confirmed that, in the experimental group (group into which collagen was injected), satisfaction with daily life was increased to 9.50 ± 12.97 after 4 weeks, 10.20 ± 16.62 after 12 weeks, and 11.79 ± 17.57 after 24 weeks in comparison with the case before treatment (C).

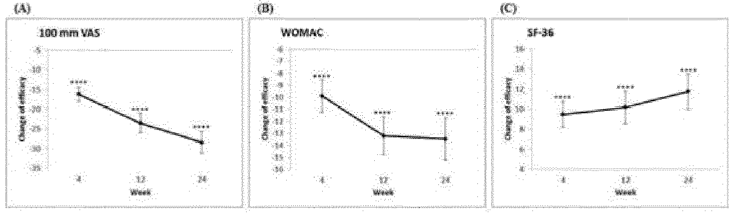

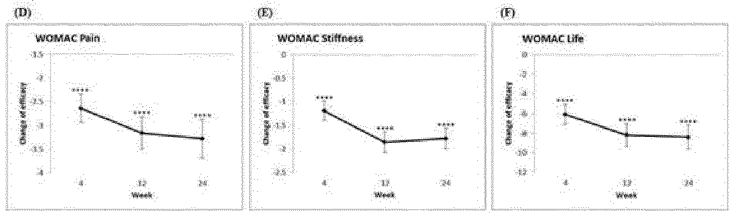

### <Results of analysis of treatment effect on experimental group using VAS, WOMAC, and SF-36 methods>

(A) 100 mm VAS analysis result, (B) WOMAC analysis result, (C) SF-36 analysis result
(D) WOMAC pain analysis result, (E) WOMAC stiffness analysis result, (F) WOMAC quality of life

### Industrial Applicability

The technical idea of the method of manufacturing collagen for restoring cartilage tissue according to the present invention and the usage method thereof make it possible to repeatedly ensure the same result. In particular, the present invention can promote technical development, thus contributing to industrial development, so it is worth protecting.

## Claims

1. A method of manufacturing collagen for restoring a cartilage tissue, in which a pig skin tissue is fragmentized, washed, and subjected to enzyme treatment, a collagen is separated therefrom, and high-concentration collagen aseptically loaded into an injection container is obtained through salt treatment, sterilization by filtration, concentration, and filling processes, the method comprising:
a process of separating the collagen from the pig skin tissue and filling the injection container with the collagen, the process comprising:
washing the skin tissue separated from a pig using ethanol and sodium hydroxide, followed by fragmentizing;
mixing the fragmentized tissue with an acidic solution containing an enzyme, followed by agitation to perform a reaction;
adding NaCl in order to obtain the collagen separated from the tissue;
performing passing through a membrane filter to separate collagen molecules, followed by passing through a filter to obtain the sterile collagen;
performing agglomeration of the collagen using a pH and a temperature, followed by concentration via centrifugation; and
filling the injection container with the concentrated collagen using sterile filling tools.

2. The method of claim 1, wherein at least 70% ethanol is used for 24 hours or less.

3. The method of claim 1, wherein sodium hydroxide having a pH of 11 or more is used for 2 hours or less.

4. The method of claim 1, wherein the fragmentized tissue is mixed with the acidic solution containing the enzyme and having a pH of 3 or less and is then agitated at 30°C or less for 3 to 5 days to perform a reaction.

5. The method of claim 1, wherein a pore size of the filter having pores is 0.22 µm or less.

6. The method of claim 1, wherein a final concentration for obtaining the collagen separated from the tissue is 0.7 to 0.9M.

7. A usage method of collagen for restoring a cartilage tissue manufactured by the method of claim 1, wherein the collagen with which an injection container is filled is usable for a joint in an injection manner in order to restore cartilage.

8. A usage method of collagen for restoring a cartilage tissue manufactured by the method of claim 1, wherein a collagen solution contained in an injection container is connected to an injection needle, and the injection needle is inserted into an application site without surgically incising the application site so that the collagen for restoring cartilage is injected into a joint.
